# EUROPÄISCHE PATENTANMELDUNG

(11) **EP 3 162 290 A1**
(43) Veröffentlichungstag der Anmeldung: **03.05.2017**
(21) Anmeldenummer: 16191079.9
(22) Anmeldetag: 28.09.2016
(51) Int. Cl.: A61B 6/02, A61B 6/03, A61B 6/00

(54) **TOMOGRAPHIEANLAGE UND VERFAHREN FÜR GROSSVOLUMIGE AUFNAHMEN**

(30) Priorität: 02.11.2015 DE 102015221418
(71) Anmelder: Siemens Healthcare GmbH, 91052 Erlangen (DE)
(72) Erfinder: Divoky, Robert, 91301 Forchheim (DE)

(57) **Zusammenfassung**

Die Erfindung betrifft eine Tomographieanlage (R) mit einer ersten Strahlquelle (Q1) und einem ersten Detektor (RD1), der der ersten Strahlquelle (Q1) zugeordnet ist, sowie mit einer zweiten Strahlquelle (Q2) und einem zweiten Detektor (RD2), der der zweiten Strahlquelle (Q2) zugeordnet ist. Die Tomographieanlage (R) ist dazu vorbereitet, einen Scan durchzuführen, wobei der erste Detektor (RD1) in einer ersten Rotationsebene (RE1) entlang einer ersten kreissegmentförmigen Bahnkurve (BKD1) geführt wird, während der zweite Detektor (RD2) synchron in einer zweiten Rotationsebene (RE2) entlang einer zweiten kreissegmentförmigen Bahnkurve (BKD2) geführt wird. Außerdem ist die Tomographieanlage (R) dazu vorbereitet, hierbei mit dem ersten Detektor (RD1) einen ersten Datensatz (DS1) und mit dem zweiten Detektor (RD2) einen zweiten Datensatz (DS2) zu gewinnen. Die beiden Rotationsebenen (RE1, RE2) sind zueinander beabstandet angeordnet.

Die Erfindung betrifft auch ein entsprechendes Verfahren (100) zum Betreiben einer Tomographieanlage (R).

## Beschreibung

Die Erfindung betrifft eine Tomographieanlage mit einer ersten Strahlquelle und einem ersten Detektor, der der ersten Strahlquelle zugeordnet ist, sowie einer zweiten Strahlquelle und einem zweiten Detektor, der der zweiten Strahlquelle zugeordnet ist. Die Tomographieanlage ist dazu vorbereitet, einen Scan durchzuführen, wobei der erste Detektor in einer ersten Rotationsebene entlang einer ersten kreissegmentförmigen Bahnkurve geführt wird, während der zweite Detektor synchron in einer zweiten Rotationsebene entlang einer zweiten kreissegmentförmigen Bahnkurve geführt wird. Die Tomographieanlage ist dazu vorbereitet, hierbei mit dem ersten Detektor einen ersten Datensatz und mit dem zweiten Detektor einen zweiten Datensatz zu gewinnen.

Außerdem betrifft die Erfindung ein entsprechendes Verfahren zum Betreiben einer Tomographieanlage.

Jeder der beiden Datensätze ist typischerweise drei- oder vierdimensional. Unter einem dreidimensionalen Datensatz wird hier vorzugsweise ein Datensatz von einem Volumenscan verstanden, aus dem sich ein vollständiges Volumenbild rekonstruieren lässt. Unter einem vierdimensionalen Datensatz wird üblicherweise eine Folge von mindestens zwei dreidimensionalen Datensätzen verstanden, aus der sich mindestens zwei Volumenbilder rekonstruieren lassen, die zeitlich aufeinanderfolgen. Hierbei zeigt ein erstes der Volumenbilder beispielsweise eine Anflutungsphase und ein zweites der Volumenbilder beispielsweise eine Ausflussphase. Die Tomographieanlage kann beispielsweise eine Röntgen-Tomographieanlage oder eine Fluoreszenz-Tomographieanlage sein.

Typischerweise haben beide Bahnkurven eine identische Form (allerdings typischerweise mit einem gegenseitigen Versatz um eine Umfangswinkeldifferenz). Auch bleibt typischerweise ein Abstand der ersten Strahlquelle zu dem ersten Detektor konstant, während der erste Detektor entlang der kreissegmentförmigen ersten Bahnkurve um die Orbitalachse geführt wird. Dies gilt auch für einen Abstand zwischen der zweiten Strahlquelle und dem zweiten Detektor. Typischerweise ist der Abstand zwischen der zweiten Strahlquelle und dem zweiten Detektor gleich groß wie ein Abstand zwischen der ersten Strahlquelle und dem ersten Detektor. Unter Umständen ist es auch von Vorteil, wenn die beiden Abstände unterschiedlich groß sind, beispielsweise um einen Unterschied in Detektorgrößen auszugleichen. Die erste Strahlquelle und der erste Detektor können an einem gemeinsamen beweglichen Träger, beispielsweise einem selben ersten C-Bogen oder an unterschiedlichen beweglichen Trägern, beispielsweise an je einem Roboterarm befestigt sein. Entsprechendes gilt auch für die zweite Strahlquelle und den zweiten Detektor. Optional kann an dem ersten Detektor ein Bildverstärker angeschlossen sein. Dies gilt auch für die zweite Strahlquelle und den zweiten Detektor. Eine davon unabhängige Option sieht vor, dass der erste Detektor einen Bildverstärker umfasst und/oder der zweite Detektor einen Bildverstärker umfasst.

Insbesondere in der Diagnostik und Therapie werden immer höhere Anforderungen an die Leistungsfähigkeit medizinischer Geräte gestellt. Damit wird insbesondere das Ziel verfolgt, Gesundheitsgefahren und Personenschäden infolge fehlerhafter Diagnose oder Behandlung zu vermeiden.

Die DE 10 2006 040 934 A1 beschreibt ein Verfahren zum Darstellen von Arterien und/oder Venen eines Gefäßsystems mittels eines C-Bogen-Biplan-Systems, das zwei C-Bögen umfasst. Während eines Füllungslaufs wird je C-Bogen eine Sequenz von Röntgenbildern aus unterschiedlichen Projektionswinkeln aufgenommen. Die Röntgenbilder des Füllungslaufs von dem ersten und dem zweiten C-Bogen aus einer arteriellen Phase werden zu einem ersten Datensatz kombiniert. Die Rekonstruktion zu einem dreidimensionalen Bilddatensatz kann vor der Kombination der Daten der Röntgenbilder der beiden C-Bögen oder am Datensatz des extrahierten arteriellen Gefäßsystems erfolgen.

Es eine Aufgabe der vorliegenden Erfindung, eine Tomographieanlage und ein Verfahren zum Betreiben einer Tomographieanlage bereitzustellen, mit der bzw. mit dem in einem Arbeitsgang (also beispielsweise nach nur einer einzigen Kontrastmittelinjektion) eine großvolumigere 3D-Aufnahme (beispielsweise von einer Wirbelsäule, einer Aorta oder einem anderen Blutgefäß) durchgeführt werden kann als mit der bekannten Tomographieanlage.

Diese Aufgabe wird erfindungsgemäß durch eine Tomographieanlage gelöst, die Folgendes aufweist: eine erste Strahlquelle und einen ersten Detektor, der der ersten Strahlquelle zugeordnet ist, sowie eine zweite Strahlquelle und einen zweiten Detektor, der der zweiten Strahlquelle zugeordnet ist. Die Tomographieanlage ist dazu vorbereitet, einen Scan durchzuführen, wobei der erste Detektor in einer ersten Rotationsebene entlang einer ersten kreissegmentförmigen Bahnkurve geführt wird, während der zweite Detektor synchron in einer zweiten Rotationsebene entlang einer zweiten kreissegmentförmigen Bahnkurve geführt wird. Außerdem ist die Tomographieanlage dazu vorbereitet, hierbei mit dem ersten Detektor einen ersten Datensatz und mit dem zweiten Detektor einen zweiten Datensatz zu gewinnen. Die beiden Rotationsebenen sind zueinander beabstandet angeordnet.

Das erfindungsgemäße Verfahren zum Betreiben einer Tomographieanlage umfasst folgende Handlungen. Es wird ein Scan durchgeführt, wobei ein erster Detektor in einer ersten Rotationsebene entlang einer ersten kreissegmentförmigen Bahnkurve geführt wird, während ein zweiter Detektor synchron in einer zweiten Rotationsebene entlang einer zweiten kreissegmentförmigen Bahnkurve geführt wird. Hierbei wird ein erster Datensatz mit dem ersten Detektor gewonnen und ein zweiter Datensatz mit dem zweiten Detektor gewonnen. Die beiden Rotationsebenen sind zueinander beabstandet angeordnet.

Ein Konzept der vorliegenden Erfindung kann darin gesehen werden, dass die Rotationsebenen der beiden Detektoren zueinander beabstandet sind. Hierdurch wird eine maximale räumliche Ausdehnung der Aufnahmefähigkeit der Tomographieanlage in Orbitalachsenrichtung vergrößert. Dass die Rotationsebenen der beiden Detektoren zueinander beabstandet sind, bedeutet, dass ein erster Schnittpunkt einer ersten Orbitalachse der ersten Rotationsebene mit der ersten Rotationsebene beabstandet ist zu einem zweiten Schnittpunkt einer zweiten Orbitalachse der zweiten Rotationsebene mit der zweiten Rotationsebene. Typischerweise sind die beiden Orbitalachsen identisch oder verlaufen zumindest parallel zueinander angeordnet. Die beiden Datensätze stellen zusammen einen übergreifenden Datensatz dar, aus dem ein übergreifendes Tomographiebild mittels eines der bekannten Rekonstruktionsverfahren (beispielsweise mittels eines gefilterten Rückprojektionsverfahrens nach Feldkamp, Davis, Kress) erzeugt werden kann.

Eine Ausführungsform der Tomographieanlage sieht vor, dass die Tomographieanlage dazu vorbereitet ist, vor einer Bildrekonstruktion aus beiden Datensätzen einen übergreifenden zwei-, drei- oder vierdimensionalen Datensatz zu erzeugen. Hierzu können die Daten des zweiten Datensatzes mit den Daten ersten Datensatzes im angrenzenden Bereich oder im Überlappungsbereich verglichen werden und mittels einer räumlichen und/oder zeitlichen Abbildung an die Daten des ersten Datensatzes angeglichen werden, sodass ein übergreifender Datensatz geschaffen wird, dessen Daten im Überlappungsbereich einen störungsfreien Übergang zwischen dem ersten und dem zweiten Datensatz darstellen. Die Abbildung kann beispielsweise ein räumliches und/oder zeitliches Verschieben und/oder ein Drehen um einen oder mehrere Eulerwinkel (Gier-, Nick-, Rollwinkel) und/oder ein Strecken oder Stauchen und/oder eine sonstige konforme Abbildung umfassen. Geeignete Verfahren für eine solche manuelle oder selbsttätige Anpassung zwischen zwei Datensätzen werden hier nicht beschrieben, da sie dem Fachmann unter dem Begriff 'Registrierung' bekannt sind. Eine weitere Ausführungsform der Tomographieanlage sieht vor, dass die Tomographieanlage dazu vorbereitet ist, mittels einer ersten Bildrekonstruktion aus dem ersten Datensatz ein erstes zwei-, drei- oder vierdimensionales Bild, mittels einer zweiten Bildrekonstruktion aus dem zweiten Datensatz ein zweites zwei-, drei- oder vierdimensionales Bild und aus dem ersten und zweiten Bild ein übergreifendes zwei-, drei- oder vierdimensionales Bild zu erzeugen. Hierzu können die Daten des zweiten rekonstruierten Bildes mit den Daten des ersten rekonstruierten Bildes im angrenzenden Bereich oder im Überlappungsbereich verglichen werden und mittels einer räumlichen und/oder zeitlichen Abbildung an die Daten des ersten Bildes angeglichen werden, sodass ein übergreifendes Bild geschaffen wird, dessen Daten im Überlappungsbereich einen störungsfreien Übergang zwischen dem ersten und dem zweiten Bild darstellen. Auch hier kann die Abbildung beispielsweise ein räumliches und/oder zeitliches Verschieben und/oder ein Drehen um einen oder mehrere Eulerwinkel (Gier-, Nick-, Rollwinkel) und/oder ein Strecken oder Stauchen und/oder eine sonstige konforme Abbildung umfassen. Außerdem können auch hierbei geeignete Verfahren für ein manuelles oder selbsttätiges Anpassen zwischen zwei Bildern angewendet werden, die dem Fachmann unter dem Begriff 'Registrierung' bekannt sind.

Es ist zweckmäßig, wenn die beiden Rotationsebenen abzüglich einer Überlappungsbreite um weniger als eine halbe Breite des ersten Detektors zuzüglich einer halben Breite des zweiten Detektors beabstandet sind. Die Überlappungsbreite beträgt beispielsweise zwischen 10% und 20% der halben Breite des ersten Detektors in Orbitalachsenrichtung oder der halben Breite des zweiten Detektors in Orbitalachsenrichtung. Durch ein räumliches Überlappen der durch die Detektoren erfassbaren Aufnahmebereiche kann auch unter Berücksichtigung von Toleranzen eine räumliche Lücke in der übergreifenden Gesamtaufnahme vermieden werden. Außerdem kann eine Struktur des zu untersuchenden Objekts dazu genutzt werden, die Daten des zweiten Datensatzes mit den Daten des ersten Datensatzes im Überlappungsvolumen zu vergleichen und mittels einer räumlichen und/oder zeitlichen Abbildung so an die Daten des ersten Datensatzes anzugleichen, dass ein übergreifender Datensatz geschaffen wird, dessen Daten im Überlappungsvolumen einen störungsfreien Übergang zwischen dem ersten und dem zweiten Aufnahmebereich darstellen.

Typischerweise ist die zweite kreissegmentförmige Bahnkurve konzentrisch zu der ersten kreissegmentförmigen Bahnkurve angeordnet. Unabhängig davon ist auch bevorzugt, wenn ein Radius der ersten kreissegmentförmigen Bahnkurve gleich groß ist wie ein Radius der zweiten kreissegmentförmigen Bahnkurve. Beides sind Voraussetzungen dafür, dass die äußeren Abmessungen des rekonstruierbaren Volumens des übergreifenden Datensatzes stufenfrei sind.

Eine besonders zweckmäßige Ausführungsform sieht vor, dass eine Ausgangsposition der zweiten kreissegmentförmigen Bahnkurve gegenüber einer Ausgangsposition der ersten kreissegmentförmigen Bahnkurve in Rotationsrichtung um eine Umfangswinkeldifferenz versetzt angeordnet ist. Hierdurch können die beiden Detektoren Bahnkurven durchlaufen, die einen gleichen Durchmesser aufweisen und zueinander konzentrisch sind.

Bekannte Vorteile sind gegeben, wenn die Tomographieanlage dazu vorbereitet ist, mit dem ersten und/oder mit dem zweiten Detektor einen Short Scan durchzuführen. Bei einem Short Scan wird in der jeweiligen Rotationsebene eine angulare oder orbitale Rotation des jeweiligen Paars von Strahlquelle und Detektor um 180° plus einem Strahlwinkel um eine Orbitalachse des Paars durchgeführt, um einen minimalen vollständigen Datensatz für die gegebene Geometrie zu gewinnen.

Das erfassbare Aufnahmevolumen kann noch weiter vergrößert werden, wenn die Tomographieanlage dazu vorbereitet ist, mit dem ersten und/oder mit dem zweiten Detektor einen Large Volume Scan durchzuführen. Bei einem Large Volume Scan wird eine angulare oder orbitale Rotation des jeweiligen Paars von Strahlquelle und Detektor um 360° um eine Orbitalachse des Paars durchgeführt, um einen minimalen vollständigen Datensatz für die gegebene Geometrie zu gewinnen, wobei ein Mittelpunkt einer Sensorfläche des Detektors gegenüber einem Zentralstrahl des Strahlenbündels der Strahlquelle um eine halbe Detektorbreite in Rotationsrichtung verschoben ist. Dadurch wird ein Durchmesser des auswertbaren Aufnahmebereichs in Rotationsrichtung ungefähr um den Faktor zwei vergrößert.

Alternativ oder zusätzlich zu dem Large Volume Scan kann eine Ausdehnung des Sichtfeldes in Rotationsrichtung auch mittels eines Verringerns des Quelle-zu-Sensor-Abstands erreicht werden.

Ausführungsformen der Tomographieanlage können sich darin unterscheiden, dass der erste Datensatz zwei-, drei- oder vierdimensional ist. Außerdem können sich Ausführungsformen der Tomographieanlage darin unterscheiden, dass der zweite Datensatz zwei-, drei- oder vierdimensional ist. Das übergreifende rekonstruierte Bild kann ebenfalls zwei-, drei- oder vierdimensional sein, wobei dessen Dimension höchstens so groß ist, wie die Dimension desjenigen der beiden Datensätze, der die kleinere Dimension aufweist.

Die Erfindung ist anhand der beigefügten Zeichnungen näher erläutert, in denen zeigen:
- FIG 1: schematisch, perspektivisch und nicht maßstäblich eine Biplan-Tomographieanlage mit einer Patientenauflage und zwei C-Armen, und
- FIG 2: schematisch, perspektivisch und nicht maßstäblich die Patientenauflage und eine Geometrie einer mit der Biplan-Tomographieanlage durchführbaren Aufnahme eines übergreifenden Datensatzes oder Rekonstruktion eines übergreifenden Volumenbilds, und
- FIG 3: schematisch einen Ablauf eines Verfahrens zum Betreiben einer Tomographieanlage.

Die nachfolgend näher geschilderten Ausführungsbeispiele stellen bevorzugte Ausführungsformen der vorliegenden Erfindung dar.

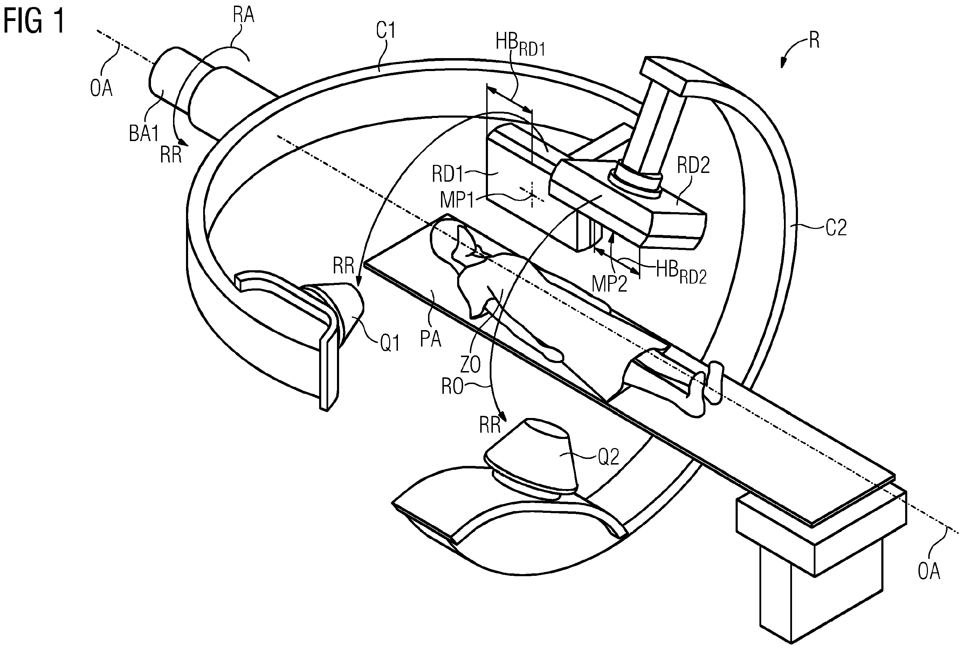

Die in FIG 1 gezeigte Biplan-Tomographieanlage R weist einen ersten C1 und einen zweiten C2 C-Bogen sowie eine Patientenauflage PA auf. An dem ersten C-Bogen C1 ist eine erste Strahlquelle Q1 und ein erster Detektor RD1 befestigt. An dem zweiten C-Bogen C2 ist eine zweite Strahlquelle Q2 und ein zweiter Detektor RD2 befestigt. Zum Durchführen eines übergreifenden Scans an einem zu untersuchenden Objekt ZO führt der erste C-Bogen C1 eine angulare Rotation RA um eine Orbitalachse OA durch, während der zweite C-Bogen C2 eine orbitale Rotation RO um dieselbe Orbitalachse OA ausführt. Bei der orbitalen Rotation RO des zweiten C-Arms C2 bleibt die (gedachte) Ebene, in der sich der zweite C-Arm C2 befindet, unverändert. Bei der angularen Rotation RA des ersten C-Arms C1 dreht sich der erste C-Arm C1 um eine Befestigungsachse BA1 des C-Arms. Grundsätzlich ist es auch möglich, einen übergreifenden Scan durchzuführen, in dem die C-Arme C1, C2 vor dem Scan so angeordnet sind, dass beide C-Arme C1, C2 beim Scan eine angulare Rotation ausführen oder dass sie vor dem Scan so angeordnet sind, dass beide C-Arme C1, C2 beim Scan eine orbitale Rotation ausführen.

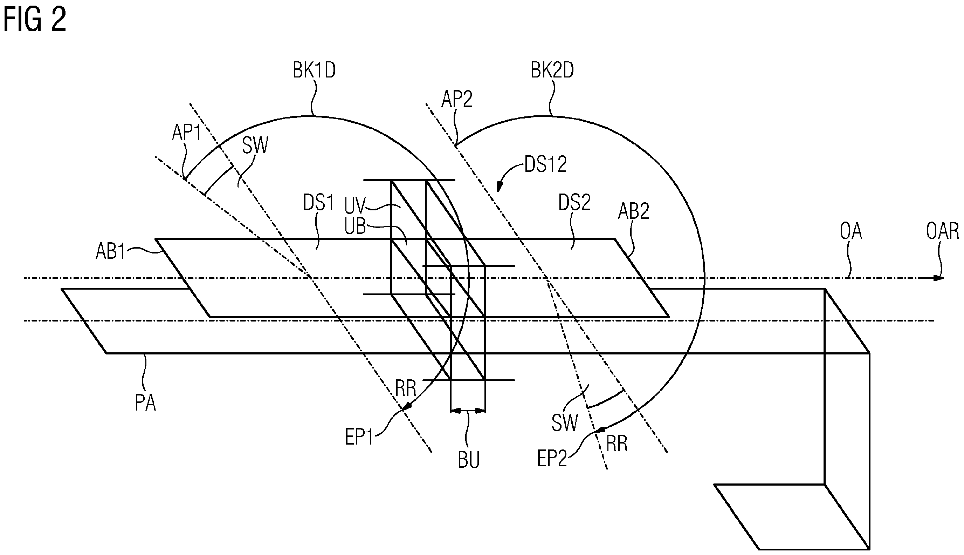

Die in FIG 2 gezeigte Geometrie einer mit der Biplan-Tomographieanlage durchführbaren Aufnahme eines übergreifenden Datensatzes oder Rekonstruktion eines übergreifenden Volumenbilds zeigt eine erste kreissegmentförmige Bahnkurve BK1D mit einer Ausgangsposition AP1 und einer Endposition EP1 eines Mittelpunkts MP1 des ersten Detektors RD1. Außerdem zeigt die Figur eine zweite kreissegmentförmige Bahnkurve BK2D mit einer Ausgangsposition AP2 und einer Endposition EP2 eines Mittelpunkts MP2 des zweiten Detektors RD2. Darüberhinaus zeigt die Figur schematisch eine räumliche Lage eines Aufnahmebereichs AB1 eines ersten Datensatzes DS1, der beim Scan mit dem ersten Detektor RD1 erfasst wird, und eine räumliche Lage eines Aufnahmebereichs AB2 eines zweiten Datensatzes DS2, der beim Scan mit dem zweiten Detektor RD2 erfasst wird. Je nach Anwendung, zu untersuchendem Körperorgan und Gefäßstruktur kann eher ein Scan im Portrait-Modus oder eher ein Scan im Landscape-Modus zweckmäßig sein. Im Portrait-Modus verläuft die längere Hauptlängsachse des Detektors parallel zur Orbitalachse, während sie im Landscape-Modus senkrecht zur Orbitalachse verläuft.

Die beiden Aufnahmebereiche AB1, AB2 überlappen sich räumlich in einem gemeinsamen Überlappungsbereich UB. Wenn die beiden Datensätze DS1, DS2 drei- oder vierdimensional sind, ist der gemeinsame Überlappungsbereich ein Überlappungsvolumen UV, das in der Figur ebenfalls schematisch dargestellt ist. Typischerweise ist die Überlappung nur in Richtung der Orbitalachse teilweise, aber in allen anderen Dimensionen vollständig. Dies gilt unabhängig davon, ob die Datensätze zwei-, drei- oder vierdimensional sind. Wenn eine möglichst vollständige Überlappung in den übrigen Dimensionen erwünscht ist, kann dies unter anderem dadurch erreicht werden, dass die Detektoren RD1, RD2 für den synchronen Scan entweder beide in einen Portrait-Modus oder beide in einen Landscape-Modus ausgerichtet sind. Es gibt Anwendungsfälle, in denen ein übergreifender Scan mit unterschiedlichen Modi zweckmäßig ist, also ein Scan im Landscape-Modus mit einem Scan im Portrait-Modus kombiniert wird (Beispiele: Kopf im Landscape-Modus und obere Wirbelsäule im Portrait-Modus; Becken im Landscape-Modus und untere Wirbelsäule im Portrait-Modus).

Eine Ausführungsform sieht vor, dass vor einer Bildrekonstruktion aus beiden Datensätzen DS1, DS2 ein übergreifender zwei-, drei- oder vierdimensionaler Datensatz DS12 erzeugt wird. Hierzu können die Daten des zweiten Datensatzes DS2 mit den Daten ersten Datensatzes DS1 im angrenzenden Bereich, im Überlappungsbereich UB oder im Überlappungsvolumen UV verglichen werden und mittels einer räumlichen und/oder zeitlichen Abbildung an die Daten des ersten Datensatzes DS1 angeglichen werden, sodass ein übergreifender Datensatz DS12 geschaffen wird, dessen Daten im angrenzenden Bereich, im Überlappungsbereich UB beziehungsweise im Überlappungsvolumen UV einen störungsfreien Übergang zwischen dem ersten DS1 und dem zweiten DS2 Datensatz darstellen. Geeignete Verfahren für ein solches manuelles oder selbsttätiges Anpassen zwischen zwei Datensätzen DS1, DS2 werden hier nicht beschrieben, da sie dem Fachmann unter dem Begriff 'Registrierung' bekannt sind.

Eine weitere Ausführungsform der Tomographieanlage sieht vor, dass die Tomographieanlage dazu vorbereitet ist, mittels einer ersten Bildrekonstruktion aus dem ersten Datensatz DS1 ein erstes zwei-, drei- oder vierdimensionales Bild, mittels einer zweiten Bildrekonstruktion aus dem zweiten Datensatz DS2 ein zweites zwei-, drei- oder vierdimensionales Bild und aus dem ersten und zweiten Bild ein übergreifendes zwei-, drei- oder vierdimensionales Bild zu erzeugen. Hierzu können die Daten des zweiten rekonstruierten Bildes mit den Daten des ersten rekonstruierten Bildes im angrenzenden Bereich, im Überlappungsbereich UB oder im Überlappungsvolumen UV verglichen werden und mittels einer räumlichen und/oder zeitlichen Abbildung an die Daten des ersten Bildes angeglichen werden, sodass ein übergreifendes Bild geschaffen wird, dessen Daten an einer Nahtstelle zwischen dem ersten und dem zweiten Bild beziehungsweise im Überlappungsbereich UB oder Überlappungsvolumen UV einen störungsfreien Übergang zwischen dem ersten und dem zweiten Bild darstellen. Auch hierbei können geeignete Verfahren für ein manuelles oder selbsttätiges Anpassen zwischen zwei Bildern angewendet werden, die dem Fachmann unter dem Begriff 'Registrierung' bekannt sind.

Es ist zweckmäßig, wenn die beiden Rotationsebenen RE1, RE2 abzüglich einer Überlappungsbreite BU um weniger als eine halbe Breite HB_{RD1} des ersten Detektors RD1 zuzüglich einer halben Breite HB_{RD2} des zweiten Detektors RD2 beabstandet sind. Die Überlappungsbreite BU beträgt beispielsweise zwischen 10% und 20% der halben Breite HB_{RD1} des ersten Detektors RD1 in Orbitalachsenrichtung OAR oder der halben Breite HB_{RD2} des zweiten Detektors RD2 in Orbitalachsenrichtung OAR. Durch ein räumliches Überlappen der durch die Detektoren RD1, RD2 erfassbaren Aufnahmebereiche AB1, AB2 kann in der übergreifenden Gesamtaufnahme auch unter Berücksichtigung von Toleranzen eine räumliche Lücke vermieden werden. Außerdem kann eine Struktur des zu untersuchenden Objekts ZO dazu genutzt werden, die Daten des zweiten Datensatzes DS2 mit den Daten ersten Datensatzes DS1 im Überlappungsvolumen UB oder im Überlappungsvolumen UV zu vergleichen und mittels einer räumlichen und/oder zeitlichen Abbildung so an die Daten des ersten Datensatzes DS1 anzugleichen, dass ein übergreifender Datensatz DS12 geschaffen wird, dessen Daten im Überlappungsvolumen UB beziehungsweise Überlappungsbereich UV einen störungsfreien Übergang zwischen dem ersten AB1 und dem zweiten AB2 Aufnahmebereich darstellen.

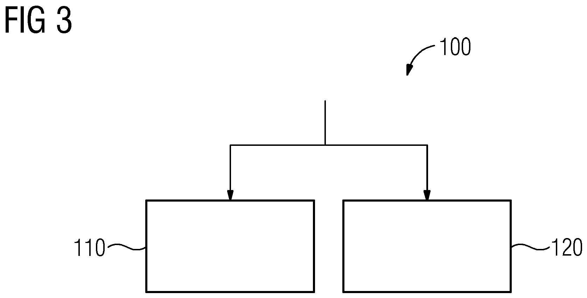

Das in FIG 3 dargestellte Verfahren 100 zum Betreiben einer Tomographieanlage R umfasst Folgende Handlungen. In einer ersten Handlung 110 wird ein Scan durchgeführt, wobei ein erster Detektor RD1 in einer ersten Rotationsebene RE1 entlang einer ersten kreissegmentförmigen Bahnkurve BK1D geführt wird, während ein zweiter Detektor RD2 synchron in einer zweiten Rotationsebene RE2 entlang einer zweiten kreissegmentförmigen Bahnkurve BKD2 geführt wird. In einer zweiten Handlung 120 wird ein erster Datensatz DS1 mit dem ersten Detektor RD1 und ein zweiter Datensatz DS2 mit dem zweiten Detektor RD2 gewonnen. Die beiden Rotationsebenen RE1, RE2 sind zueinander beabstandet angeordnet.

Die Erfindung betrifft eine Tomographieanlage R mit einer ersten Strahlquelle Q1 und einem ersten Detektor RD1, der der ersten Strahlquelle Q1 zugeordnet ist, sowie mit einer zweiten Strahlquelle Q2 und einem zweiten Detektor RD2, der der zweiten Strahlquelle Q2 zugeordnet ist. Die Tomographieanlage R ist dazu vorbereitet, einen Scan durchzuführen, wobei der erste Detektor RD1 in einer ersten Rotationsebene RE1 entlang einer ersten kreissegmentförmigen Bahnkurve BKD1 geführt wird, während der zweite Detektor RD2 synchron in einer zweiten Rotationsebene RE2 entlang einer zweiten kreissegmentförmigen Bahnkurve BKD2 geführt wird. Außerdem ist die Tomographieanlage R dazu vorbereitet, hierbei mit dem ersten Detektor RD1 einen ersten Datensatz DS1 und mit dem zweiten Detektor RD2 einen zweiten Datensatz DS2 zu gewinnen. Die beiden Rotationsebenen RE1, RE2 sind zueinander beabstandet angeordnet.

## Patentansprüche

1. Tomographieanlage (R) mit einer ersten Strahlquelle (Q1) und einem ersten Detektor (RD1), der der ersten Strahlquelle (Q1) zugeordnet ist, sowie einer zweiten Strahlquelle (Q2) und einem zweiten Detektor (RD2), der der zweiten Strahlquelle (Q2) zugeordnet ist, wobei die Tomographieanlage (R) dazu vorbereitet ist, einen Scan durchzuführen, wobei der erste Detektor (RD1) in einer ersten Rotationsebene (RE1) entlang einer ersten kreissegmentförmigen Bahnkurve (BKD1) geführt wird, während der zweite Detektor (RD2) synchron in einer zweiten Rotationsebene (RE2) entlang einer zweiten kreissegmentförmigen Bahnkurve (BKD2) geführt wird, wobei die Tomographieanlage (R) dazu vorbereitet ist, hierbei mit dem ersten Detektor (RD1) einen ersten Datensatz (DS1) und mit dem zweiten Detektor (RD2) einen zweiten Datensatz (DS2) zu gewinnen,
**dadurch gekennzeichnet, dass**
die beiden Rotationsebenen (RE1, RE2) zueinander beabstandet angeordnet sind.

2. Tomographieanlage (R) nach dem vorhergehenden Anspruch, **dadurch gekennzeichnet, dass** die Tomographieanlage (R) dazu vorbereitet ist, vor einer Bildrekonstruktion aus beiden Datensätzen (DS1, DS2) einen übergreifenden zwei-, drei- oder vierdimensionalen Datensatz (DS12) zu erzeugen.

3. Tomographieanlage (R) nach einem der vorhergehenden Ansprüche, **dadurch gekennzeichnet, dass** die Tomographieanlage (R) dazu vorbereitet ist, mittels einer ersten Bildrekonstruktion aus dem ersten Datensatz (DS1) ein erstes zwei-, drei- oder vierdimensionales Bild, mittels einer zweiten Bildrekonstruktion aus dem zweiten Datensatz (DS2) ein zweites zwei-, drei- oder vierdimensionales Bild und aus dem ersten und dem zweiten Bild ein übergreifendes zwei-, drei- oder vierdimensionales Bild zu erzeugen.

4. Tomographieanlage (R) nach einem der vorhergehenden Ansprüche, **dadurch gekennzeichnet, dass** die beiden Rotationsebenen (RE1, RE2) abzüglich einer Überlappungsbreite (BU) um weniger als eine halbe Breite (HB_{RD1}) des ersten Detektors (RD1) zuzüglich einer halben Breite (HB_{RD2}) des zweiten Detektors (RD2) beabstandet sind.

5. Tomographieanlage (R) nach einem der vorhergehenden Ansprüche, **dadurch gekennzeichnet, dass** die zweite kreissegmentförmige Bahnkurve (BKD2) konzentrisch zu der ersten kreissegmentförmigen Bahnkurve (BKD1) angeordnet ist und/oder dass ein Radius der ersten kreissegmentförmigen Bahnkurve (BKD1) gleich groß ist wie ein Radius der zweiten kreissegmentförmigen Bahnkurve (BKD2).

6. Tomographieanlage (R) nach einem der vorhergehenden Ansprüche, **dadurch gekennzeichnet, dass** eine Ausgangsposition (AP2) der zweiten kreissegmentförmigen Bahnkurve (BKD2) gegenüber einer Ausgangsposition (AP1) der ersten kreissegmentförmigen Bahnkurve (BKD1) in Rotationsrichtung (RR) um eine Umfangswinkeldifferenz versetzt angeordnet ist.

7. Tomographieanlage (R) nach einem der vorhergehenden Ansprüche, **dadurch gekennzeichnet, dass** die Tomographieanlage (R) dazu vorbereitet ist, mit dem ersten (RD1) und/oder mit dem zweiten (RD2) Detektor einen Short Scan durchzuführen.

8. Tomographieanlage (R) nach einem der vorhergehenden Ansprüche, **dadurch gekennzeichnet, dass** die Tomographieanlage (R) dazu vorbereitet ist, mit dem ersten (RD1) und/oder mit dem zweiten (RD2) Detektor einen Large Volume Scan durchzuführen.

9. Tomographieanlage (R) nach einem der vorhergehenden Ansprüche, **dadurch gekennzeichnet, dass** der erste Datensatz (DS1) zwei-, drei- oder vierdimensional ist und/oder dass der zweite Datensatz (DS2) zwei-, drei- oder vierdimensional ist.

10. Verfahren (100) zum Betreiben einer Tomographieanlage (R), wobei das Verfahren (100) folgende Handlungen umfasst:
- Durchführen (110) eines Scans, wobei ein erster Detektor (RD1) in einer ersten Rotationsebene (RE1) entlang einer ersten kreissegmentförmigen Bahnkurve (BKD1) geführt wird, während ein zweiter Detektor (RD2) synchron in einer zweiten Rotationsebene (RE2) entlang einer zweiten kreissegmentförmigen Bahnkurve (BKD2) geführt wird, wobei die beiden Rotationsebenen (RE1, RE2) zueinander beabstandet angeordnet sind,
- Gewinnen (120) eines ersten Datensatzes (DS1) mit dem ersten Detektor (RD1) und Gewinnen eines zweiten Datensatzes (DS2) mit dem zweiten Detektor (RD2).
